# EUROPEAN PATENT SPECIFICATION

(11) **EP 2 292 727 B1**
(45) Date of publication and mention of the grant of the patent: **23.10.2013**
(21) Application number: 09770037.1
(22) Date of filing: 08.06.2009
(51) Int. Cl.: C12M 1/00, C12Q 1/68, G01N 21/78, G01N 37/00

(54) **SINGLE MOLECULE REAL TIME SEQUENCER, NUCLEIC ACID ANALYZER AND SINGLE MOLECULE REAL TIME SEQUENCING METHOD**
EINZELMOLEKÜL-ECHTZEIT-SEQUENZIERGERÄT, NUKLEINSÄURE-ANALYSEGERÄT UND EINZELMOLEKÜL-ECHTZEIT-SEQUENZIERVERFAHREN
SÉQUENCEUR EN TEMPS RÉEL DE MOLÉCULES SIMPLES, ANALYSEUR D'ACIDES NUCLÉIQUES ET PROCÉDÉ DE SÉQUENÇAGE EN TEMPS RÉEL DE MOLÉCULES SIMPLES

(30) Priority: 23.06.2008 JP 2008162667
(43) Date of publication of application: 09.03.2011
(73) Proprietor: Hitachi High-Technologies Corporation, Tokyo 105-8717 (JP)
(72) Inventor: KATO, Hirokazu, Ibaraki 312-8504 (JP); TAKAHASHI, Satoshi, Ibaraki 312-8504 (JP); KUMAZAKI, Nobutaka, Ibaraki 312-8504 (JP); HAGA, Takanobu, Tokyo (JP)
(74) Representative: Strehl Schübel-Hopf & Partner
(86) International application number: PCT/JP2009/060808
(87) International publication number: WO 2009/157327

(56) References cited:
- WO-A1-98/33939
- WO-A2-2005/080605
- WO-A2-2006/083751
- WO-A2-2007/147110
- JP-A- 2004 219 369
- JP-A- 2006 003 217
- JP-A- 2006 308 332
- JP-A- 2007 295 836
- RUPAREL H ET AL: "Design and synthesis of a 3 '-O-allyl photocleavable fluorescent nucleotide as a reversible terminator for DNA sequencing by synthesis", PROCEEDINGS OF THE NATIONAL ACADEMY OF SCIENCES, NATIONAL ACADEMY OF SCIENCES, WASHINGTON, DC; US, vol. 102, no. 17, 1 April 2005 (2005-04-01), pages 5932-5937, XP002454222, ISSN: 0027-8424, DOI: 10.1073/PNAS.0501962102
- LI ZENGMIN ET AL: "A photocleavable fluorescent nucleotide for DNA sequencing and analysis", PROCEEDINGS OF THE NATIONAL ACADEMY OF SCIENCES, NATIONAL ACADEMY OF SCIENCES, WASHINGTON, DC; US, vol. 100, no. 2, 21 January 2003 (2003-01-21), pages 414-419, XP002408035, ISSN: 0027-8424, DOI: 10.1073/PNAS.242729199
- METZKER MICHAEL L: "APPLICATIONS OF NEXT-GENERATION SEQUENCING Sequencing technologies - the next generation", NATURE REVIEWS GENETICS, vol. 11, no. 1, January 2010 (2010-01), pages 31-46, XP000002659622, ISSN: 1471-0056

## Description

### Technical Field

The present invention relates to an analysis method for a nucleic acid sequence and an analyzer of the nucleic acid sequence. More specifically, the present invention relates to a method and a device to decode a base sequence of, for example, a nucleic acid such as DNA or RNA.

### Background Art

A human genome plan, where 3 billion dollars of budget has been spent from 1990 to 2005, enabled to read a part easiest to decode (93% of the whole parts) 2 to 3 years earlier than originally planned, and has left, as a property, a technology or a method required in decoding, as shown in the Non Patent Literature 1. Such technology has shown further improvement thereafter, and at present, genome decoding becomes possible in precision practically endurable in a cost of about 20 million dollars ($2×10⁷). Decoding of a large scale base sequence in this high cost is still limited only by a special decoding center, or a research project obtained a high amount of budget. However, cost reduction in sequence determination will enable to handle much larger quantity of genome by many parties. For example, it becomes possible to compare genomes of a patient and a healthy person, and as a result, enhancement of value of genome information is expected. As shown in the Non Patent Literature 2, acquisition of such a fundamental data is expected to largely contribute to development of personalized medical treatment in the future.

Under such circumstances, two programs for "Innovative genome sequence determination technology", under financial support by The National Institutes of Health (NIH), aim at human genome decoding by 100 thousand dollars ($1×10⁵) for one person till 2009, and by thousand dollars ($1×10³) till 2014, namely, development of what is called "thousand dollars genome" decoding technology.

As shown in the Non Patent Literature 3, there have been commercialized already several next generation sequencers. These technologies have already attained 1/10 to 1/100 level cost as compared with conventional technology. In addition, number of bases measurable by one time analysis has attained an order of 10⁹. However, enhancement of these sequencers in view of cost has nearly saturated, and it is estimated that attainment of the "thousand dollars genome" by a device and a system already commercialized is difficult.

In addition, the next generation sequencers now available on the market are capable of decoding a base sequence of up to about 10⁹, however, they require running time of 2 to 3 days only for sequence decoding of a data volume as large as about 10⁹. In a practical medical field, for genome sequence decoding on an individual basis to be a routine work, not only cost reduction but also high speed sequence decoding are necessary.

One promising sequence technology, expected under these circumstances, is a single molecule real time sequencing method, namely, a method for measuring by real time directly in a single molecule level, at a field of an enzyme reaction. The single molecule real time sequencing method carries out fluorescence detection in real time where base extension is carried out. Therefore, it is capable of carrying out base sequence decoding in dramatically high speed. In addition, in the single molecule real time sequencing method, amplification of a sample by PCR is not required, and thus dramatic cost reduction is also possible. Therefore, the single molecule real time sequencing method is one of the most promising technologies for attaining the "thousand dollars genome".

The Non Patent Literature 4 has disclosed one of the single molecule real time sequencing methods. In this Literature, a measurement method is adopted for energy fluorescence transfer to acceptor-nucleotide accompanying with the extension reaction of nucleic acid, by immobilization of polymerase modified with a donor fluorescence body on a substrate.

In addition, as technology for controlling various biological processes proceeding in a cell, and analyzing it in real time, a caged compound can be included. The caged compound is a generic term for one that modifies a biologically active molecule by a photodegradable protecting group to deactivate temporarily. It has a name of the "caged compound" to mean a molecule to have biological activity slept by putting it in a cage. By irradiation of light with an optimal wavelength for the compound onto the caged compound, the protecting group is dislocated only at the site irradiated in an instant of light irradiation, resulting in expression of original biological activity. That is, it becomes possible to control temporal and spatial dynamics of a chemical reaction by irradiation of light. In the Patent Literatures 1 and 2, the caged compound is used to prepare a pool of oligo nucleotides. However, this method uses a sequential reaction, and is a method for sequentially decomposing the caged compound bound to the terminal, accompanying with the reaction. Therefore, this method is not used for real time analysis. In addition, a target of this method is multi-molecules and not a single molecule.

### Citation List

### Patent Literatures

Patent Literature 1: JP-A-2006-503586
Patent Literature 2: US 2007/0059692

### Non Patent Literatures

Non Patent Literature 1: Nature Reviews, Vol. 5, pp 335, 2004.
Non Patent Literature 2: "From complete decoding of human genome to understanding of "human", pp 253, Shohei Hattori, Toyo Shoten, 2005.
Non Patent Literature 3: Nature, Vol. 449, pp 627, 2007.
Non Patent Literature 4: "Next generation Sequencing: Scientific and Commercial Implications of the $ 1,000 genome", Kevin Davies, pp 41, Insight Pharma Reports.
Non Patent Literature 5: Anal. Chem. Vol. 78, 6238-6245.
Non Patent Literature 6: Nanotechnology, 2007, vol. 18, pp 44017-44021.
Non Patent Literature 7: Nano Letters. 2004, vol. 4, 957-961.

WO 2007/147110 A2 and WO 2005/080605 A2 disclose nucleic acid analyzers related to the one of the present invention but required to involve sensitive imaging sensors in order to process signals with low signal-to-noise ratio.

### Summary of Invention

### Technical Problem

As a problem of the single molecule real time sequencing method, control of initiation of the extension reaction is included. In general, in the extension reaction, fluorescence from a single molecule immobilized on a substrate is detected with a CCD camera through an objective lens. However, because the objective lens has limited observable view, it cannot observe the whole surface of substrate at one time. On the other hand, in a real time reaction, the extension reaction is initiated with pouring a reaction solution. Therefore, it is necessary to initiate the extension reaction only at the substrate area observable with the objective lens, and establish a reaction system where the reaction does not proceed at other areas. In order to attain this, it is considered a method for dividing a flow cell to a plurality of reaction fields, and sending a liquid to these reaction fields independently or sequentially. However, decoding of human genome requires several-hundred or more reaction fields, and thus preparation of complicated channels. In addition, to correctly send a liquid to a plurality of the reaction fields independently or sequentially, plural units of high performance pumps are required. Furthermore, to stably send the liquid to a plurality of the reaction fields independently or sequentially is predicted to be extremely difficult due to generation problems of pressure loss, air bubbles, contamination causing measurement noise and the like. Still more, the complicated channels and a plurality of pumps have drawback of increasing cost.

Accordingly, it has been required a methodology for initiating the extension reaction only at a desired area in the substrate, without preparing the complicated channels.

An object of the present invention relates to selectively control the extension reaction in a desired area in the substrate.

### Solution to Problem

The present invention is defined in the independent claims. Further advantageous features are set out in the dependent claims.

The present invention immobilizes an oligo probe arranged with a caged compound at the terminal thereof to a reaction field area in the substrate. After pouring a reaction solution into a flow cell including the reaction field area, the reaction field area alone is irradiated with light to dissociate the photodegradation-active protecting group at the terminal of the oligo probe that has been immobilized in said reaction field area to control initiation of a polymerase extension reaction selectively. In the flow cell, a plural number of the reaction field areas are arranged at constant interval on the substrate. The flow cell immobilized in a moving stage is moved by a distance equal to the interval between the adjacent reaction field areas and then light irradiation is carried out to measure the extension reaction continuously. By repeating these operations, the base extension reaction is stably measured in the flow cell without using complicated channels or without replacing the reaction solution.

### Advantageous Effects of Invention

According to the present invention, a real time base extension reaction can be measured without using a flow cell configured by complicated channels and a liquid sending mechanism. In addition, because a simple channel system can be adopted, problems such as generation of air bubbles, and liquid leakage in the channels accompanying with sending the liquid can be prevented beforehand, and also preparation cost of the flow cell can be reduced.

### Brief Description of Drawings

Fig. 1 is a schematic drawing showing a fundamental configuration of a flow cell structure.
Fig. 2 is an explanation drawing on a control method for a real time base extension reaction.
Fig. 3 is an explanation drawing on a chemical structure of an oligo probe.
Fig. 4 is an explanation drawing on an device configuration using an extension reaction control method by light irradiation.
Fig. 5 is a flow diagram of a real time extension reaction.

### Description of Embodiments

An Embodiment discloses a single molecule real time sequencer having: a nucleic acid probe having a photodegradable substance that inhibits a nucleic acid extension reaction; a reaction field area arranged with a plurality of said nucleic acid probes; a channel installed with a plurality of said reaction field areas; a reagent supply mechanism for supplying a reagent to be utilized in the nucleic acid extension reaction to the channel; an excited optical system for irradiating the evanescent light to the desired reaction field area and detecting the fluorescence generated; and a reaction control optical system for irradiating the light to decompose the photodegradable substance to the desired reaction field area.

In addition, an Embodiment discloses a nucleic acid analyzer having: a nucleic acid analysis device having a nucleic acid probe that hybridizes with a target nucleic acid but does not promote a nucleic acid extension reaction unless UV light is irradiated, a reaction field area arranged with a plurality of the nucleic acid probes that hybridize with different target nucleic acids, and a channel that is installed with a plurality of said reaction field areas, and that is capable of retaining a reagent to be utilized in the nucleic acid reaction; an excited optical system for irradiating the evanescent light to the desired reaction field area and detecting the fluorescence generated; a reaction control optical system for irradiating UV light to decompose the photodegradable substance to the desired reaction field area; and a stage drive mechanism for moving relatively the nucleic acid analysis device to the excited optical system and the reaction control optical system, so as to irradiate evanescent light and UV light to the desired reaction field area.

In addition, an Embodiment discloses a single molecule real time sequencing method comprising: to prepare a nucleic acid probe having a photodegradable substance that inhibits a nucleic acid extension reaction, and a channel installed with a plurality of reaction field areas arranged with a plurality of said nucleic acid probes; to supply a target nucleic acid and a reagent to be utilized in the nucleic acid extension reaction, in the channel; to irradiate UV light to decompose the photodegradable substance to the desired reaction field area, to irradiate evanescent light to the desired reaction field area, and to detect fluorescence generated; to move relatively the channel to the reaction control optical system for irradiating the UV light, and the excited optical system generating evanescent light; to irradiate UV light to decompose the photodegradable substance to the different reaction field areas, to irradiate evanescent light to the different reaction field area, and to detect fluorescence generated.

In addition, an Embodiment discloses one where the photodegradable substance is a photodegradable protecting group modified at the terminal of the nucleic acid probe.

In addition, an Embodiment discloses one where the photodegradable protecting group is a 2-nitrobenzyl type, a decyl-phenacyl type or a coumarinylmethyl type.

In addition, an Embodiment discloses one where a metal structure that generates a fluorescence increasing field is arranged in the reaction area field, corresponding to each of the nucleic acid probes.

In addition, an Embodiment discloses one where the reagent contains four deoxynucleotides, dATP, dCTP, dTTP and dGTP, labeled with four-colored fluorescent dyes emitting each different fluorescence depending on the evanescent light, and an enzyme for carrying out the nucleic acid extension reaction.

In addition, an Embodiment discloses one where the excited optical system illuminates by total reflection of visible light having a wavelength range of 420 nm to 800 nm, and generates the evanescent light.

In addition, an Embodiment discloses one where the reaction control optical system irradiates UV light having a wavelength range of 250 nm to 400 nm.

In addition, an Embodiment discloses one where, after the reaction control optical system irradiates light to the desired reaction area field, the channels are moved to capture, in a view thereof, the reaction area field that has the different reaction control optical system.

In addition, an Embodiment discloses a reaction control device having a single molecule as a measurement target, a metal structure for retaining the single molecule, an area regularly arranged with the metal structure, a channel regularly arranged with the area, a substrate arranged with one or more channels, a reaction solution containing a chemical substance interacting with the single molecule, a photodegradable substance inhibiting a chemical reaction between the single molecule and the chemical substance in the reaction solution, a unit for removing the inhibition of the photodegradable substance to locally initiate the interaction between the single molecule and the reaction solution, a unit for transferring the substrate, and a unit for supplying the reaction solution to the channel.

In addition, an Embodiment discloses one where the single molecule is DNA or RNA, retention of the single molecule is one by hybridization with a complementary strand of the single molecule immobilized at the metal structure, the photodegradable substance is the photodegradable protecting group modified at the terminal of the complementary strand, a unit for removing inhibition of the photodegradable protecting group is one by irradiation of light, a chemical substance contained in the reaction solution is polymerase that is an enzyme for carrying out the extension reaction of four deoxynucleotides, dATP, dCTP, dTTP and dGTP, labeled with four-colored fluorescent dyes emitting each different fluorescence, and nucleic acid.

In addition, an Embodiment discloses one where a unit for measuring the extension reaction of the nucleic acid is fluorescence measurement by total reflected illumination using visible light having a wavelength range of 420 nm to 800 nm, characterized in that size of the metal structure is smaller than wavelength of the visible light, and one for enhancing electric field intensity at the vicinity of the metal structure, and enhancing fluorescence from the fluorescent dye, by generation of surface Plasmon resonance by irradiation of visible light onto the metal structure.

In addition, an Embodiment discloses one where the unit for removing inhibition of the photodegradable protecting group uses UV light having a wavelength range of 250 nm to 400 nm, and one having a unit for selectively irradiating the UV light only to an area regularly arranged with the structure.

In addition, an Embodiment discloses one where the unit for transferring the substrate by a distance equal to the interval between the adjacent areas, after completion of the extension reaction by selectively irradiating UV light only to the area regularly arranged with the structure, is by an XY stage, and one having a unit for continuously measuring the extension reaction at the area, and a unit for auto-focusing to a fluorescent molecule of the substrate.

In addition, an Embodiment discloses one where the above photodegradable substance is a photodegradable protecting group, and the protecting group is a 2-nitrobenzyl type, a decyl-phenacyl type or a coumarinylmethyl type.

In addition, an Embodiment discloses one where a sequence of the complementary strand immobilized at the metal structure is a polyT sequence that captures mRNA.

In addition, an Embodiment discloses one where the unit for carrying out the fluorescence detection is configured by an objective lens, a notch filter, a dichroic mirror, a bandpass filter, a condensing lens, and a CCD camera.

In addition, an Embodiment discloses one where the total reflected illumination is configured by a plurality of lasers, a mirror, an ND filter, λ/4 wavelength plate, a shutter, a condensing lens, a prism, and coupling oil.

In addition, an Embodiment discloses one where a unit for supplying the reaction solution to the channel is configured by a dispensing unit, a waste liquid tank and a scepter of the substrate.

In addition, an Embodiment discloses one where the deoxynucleotide is Alexa488-dCTP, Cy3-dATP, Cy5-dCTP and Cy5.5-dCTP.

Explanation will be given below on the above and other features and effects of the novel invention, with reference to drawings. It should be noted that, the drawings are only for understanding the present invention, and not for limiting the scope of protection as defined by the claims.

### [Embodiments]

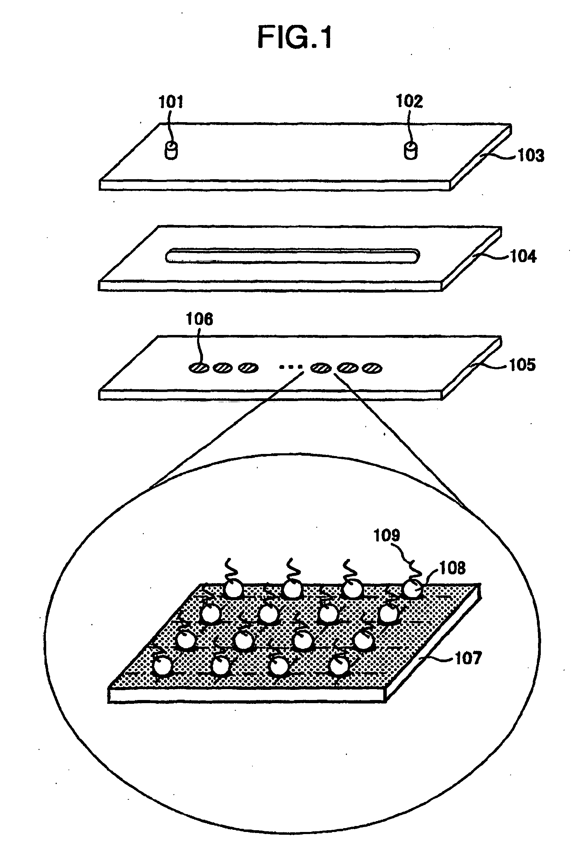

Explanation will be given below on a flow cell arranged with a plurality of reaction fields in one channel, as one Embodiment of the present invention, with reference to Fig. 1. By sandwiching PDMS 104 (polydimethylsilozane), where a rectangle-shaped space part is formed, between substrates 105 and 103 having light transmission, the flow cell is formed. PDMS is a silicone resin and is capable of forming a channel by pouring and polymerizing a not yet polymerized PDMS liquid in a mold of the channel. In addition, PDMS, because of having extremely low light absorption in a visible light region, is suitable for fluorescence measurement under a microscope. In addition, PDMS, because of having adhered to the surface of a flat and smooth substrate 103 only by van der Waals' force, adheres to the substrate only by being pushed on the surface of the substrate 103. On the substrate 103, rubber-like scepters 101 and 102 are installed for pouring a reagent such as a reaction solution, a washing liquid. By piercing an injection needle or the like into this scepter, it becomes possible to pour the desired liquid in a channel manually or automatically. Still more, on the substrate 105, 200 pieces of reaction fields 106 are arranged linearly. Interval between adjacent reaction fields 106 is 0.5 mm. In addition, the reaction field 106 is prepared in a size of 0.5 mm in diameter, nearly equal to the maximum view of the objective lens to be used.

A substrate 107 of Fig. 1 is a magnified view of the reaction field 106, and in the substrate 107, a gold structure 108 is arranged regularly in a lattice-like way in a pitch of 1 µm. In addition, on the gold structure 108, an oligo probe 109 is immobilized one piece by one piece. In particular, in the case of carrying out expression analysis of mRNA, the oligo probe 109 has a repeated sequence of dT. The gold structure 108 is a structure smaller than wavelength of excited light, and in the present Embodiment, a gold particle with a diameter of 80 nm was adopted. The gold structure 108 is arranged on the substrate 107. When exited light is irradiated on the gold structure 108, enhancement of an electric field at the vicinity of the gold structure 108 is generated by Plasmon phenomenon, and fluorescence to be measured is enhanced. Plasmon phenomenon means collective excitation of free electrons by confinement of light.

As the gold structure 108 to enhance fluorescence, there are present a pyramid shape, a shape where an insulating film is sandwiched between the two gold structured bodies (a laminated layer shape), a shape arranged with two triangle poles, like a bow tie, or the like. In the case of the pyramid shape, an oligo probe is arranged at the tip part thereof or the like. In the case of the laminated layer shape, the oligo probe is arranged in the insulating film or the like. In the case of the shape arranged with the two triangle poles, the oligo probe is arranged in the space sandwiched. In addition, as the metal body which is capable of generating localized-type surface Plasmon, gold, silver, platinum, aluminum and copper or the like is known, and as a material of the gold structure 108, in addition to gold, these metals may be used as well.

It should be noted that, as for phenomenon of fluorescence enhancement by surface Plasmon, one using an island structure of nanometer-order silver, as reported in Anal. Chem. Vol. 78, 6238-6245 (Non Patent Literature 5), or one using a gold spherical fine particle with a diameter of several-ten nanometer, as reported in Nanotechnology, 2007, vol. 18, pp 44017-44021 (Non Patent Literature 6), is known. It has been shown in Nano Letters. 2004, vol. 4, 957-961 (Non Patent Literature 7), that when the triangle poles come close, strong localized-type surface Plasmon generates in a space therebetween.

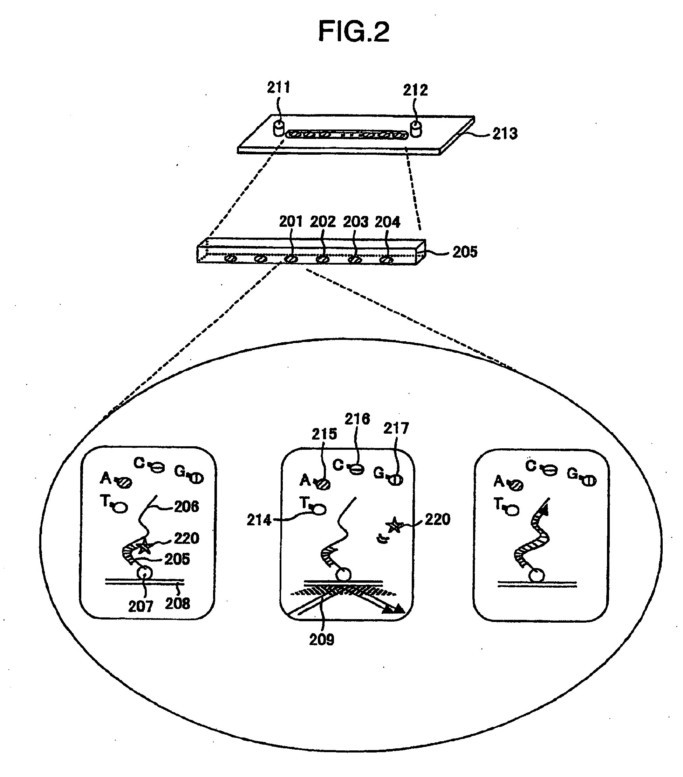

Explanation will be given next on control of a real time base extension reaction using UV light 209, with reference to Fig. 2. Inside a channel 205 in a substrate 213, a reaction solution is poured through scepters 211 and 212. The reaction solution contains four kinds of nucleotides each labeled with a different fluorescent dye, and polymerase. Each nucleotide is Alexa488-dCTP 214, Cy3-dATP 215, Cy5-dCTP 216 and Cy5.5-dCTP 217. Concentration of each nucleotide is 200 nM. In addition, in the reaction solution, slat concentration, magnesium concentration and pH are optimized so that the extension reaction is carried out efficiently.

Inside the reaction fields 201, 202, 203 and 204, as already explained in Fig. 1, a gold structure 207 is arranged regularly in a lattice-way in a pitch of 1 µm. One chain of an oligo probe 205 is covalently bonded to one gold structure 207. In addition, before initiation of the reaction, mRNA 206 whose sequence is to be decoded, and the oligo probe 205 are in a hybridized state. At 3' OH in a nucleotide at the 3' terminal of the oligo probe 205, a caged compound 220 as a photodegradation-active protecting group, is labeled. Because this caged compound 220 inhibits the extension reaction of the complementary strand of the mRNA 206 of a target nucleic acid, the extension reaction does not proceed even if the flow cell is filled with the reaction solution. However, by irradiation of UV light 209 with a wavelength of 260 nm only at the reaction field 201, the caged compound 220 at the terminal of the oligo probe on the reaction field 201 is dissociate, and the extension reaction can be started at arbitrary time.

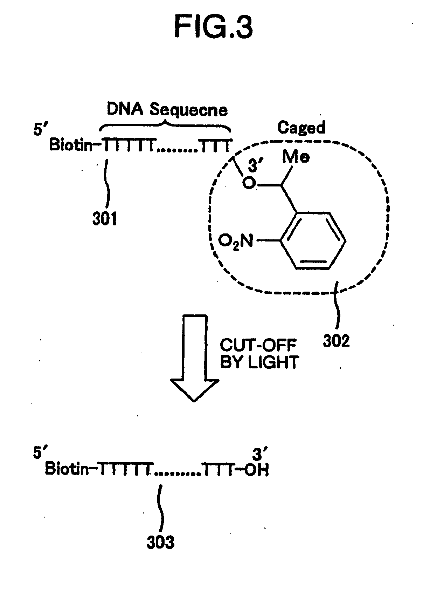

Explanation will be given on chemical structure of the above-described oligo probe, with reference to Fig. 3. An oligo probe 301 of the present Embodiment is modified with biotin at the 5' terminal. It aims at immobilizing the oligo probe 301 at the gold structure by utilization of an adipin-biotin bonding. It should be noted that, modification of the 5' terminal is not limited to biotin, and a method for utilizing a covalent bond between an amino group, a maleimide group, or a thiol group and gold can also be used.

In the present Embodiment, explanation was given on sequence analysis of mRNA, as an Embodiment, and to attain selective hybridization with a polyA part present at the 3' terminal of mRNA, a base sequence of the oligo probe 301 in the present Embodiment takes a polyT sequence.

In addition, the 3' terminal of the oligo probe 301 is modified with the photodegradative protecting group. Although various photodegradative protecting groups have been reported up to now, in the present Embodiment, a 2-nitrobenzyl type, as a typical photodegradative protecting group, was used. Optimal wavelength to activate a 2-nitrobenzyl type compound of the present Embodiment is 260 nm. After UV light irradiation, the photodegradative protecting group is deprotected, and the oligo probe 301 is converted to a molecular structure of an oligo probe 303. In the oligo probe 303, where the photodegradative protecting group is deprotected, because a substance inhibiting the extension reaction has already been dissociated, the extension reaction proceeds where the fluorescent labeled nucleotide floating in a solution is incorporated by a polymerase, occurs. By this mechanism, control of the extension reaction by light irradiation can be carried out.

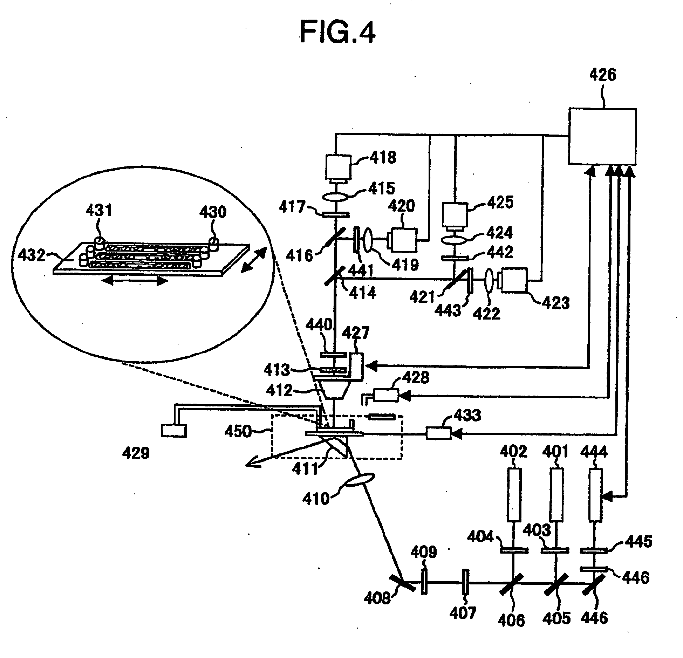

Explanation will be given below on a nucleic acid analyzer suitable for the above-described extension reaction technology, with reference to Fig. 4. In order to excite four different fluorescent dyes, a device of the present Embodiment is provided with two lasers, an Argon laser 401 with a wavelength of 514 nm, and a He-Ne laser 402 with a wavelength of 633 nm. Reason for using two lasers instead of four lasers is to reduce cost, and four lasers may be provided. The Argon laser 401 is used to excite an Alexa488-dCTP 214 and a Cy3-dATP 215 of fluorescent labeled nucleotide, while the He-Ne laser 402 is used to excite a Cy5-dCTP 216 and a Cy5.5-dCTP 217. Excited light emitted from the Argon laser 401 and the He-Ne laser 402 is reflected at a dichroic mirror 405 and a dichroic mirror 406, respectively, and injected to a bottom surface of a substrate 432 via an ND filter 407, a 4/λ wavelength plate, a mirror 408, a condenser lens 410 and a prism 411. Incident angle is equal to or larger than critical angle, and a fluorescent dye on the flow cell substrate is excited by total reflected illumination. Here, advantage of utilizing total reflected illumination is not to excite all fluorescent dyes being free in the reaction solution but to enable to attain local illumination at only an area at the vicinity of about 150 nm in depth in a z direction from the substrate. Therefore, it enables to significantly reduce back light, which becomes noise, as compared with a usual epi-illumination excitation method. Still more, excited light irradiated on the gold structure generates surface Plasmon resonance and thus can provide several-ten times of enhancement effect of fluorescence in an area with a size nearly to that of the gold structure. Fluorescence emitted reaches a dichroic mirror 414 via an objective lens 412, a notch filter 413, and a bandpass filter 440. Fluorescence going straight is still more divided by a dichroic mirror 416, each passing through a bandpass filter 417 and 441 to be focused on image screens of CCD cameras 418 and 420 by condensing lenses 415 and 419. Similarly, fluorescence reflected by the dichroic mirror 414 is still more divided by a dichroic mirror 421, each passing through a bandpass filter 442 and 443 to be focused on image screens of CCD cameras 425 and 423 by condensing lenses 424 and 422. In this way, it becomes possible to acquire simultaneously fluorescence images of four different fluorescent dyes. A signal acquired by each of CCD cameras 418, 420, 423 and 425 is processed by a control PC 426 and converted to a base sequence of mRNA.

It should be noted that, in the above Embodiment, the fluorescent dye is excited using total reflected illumination, however, the fluorescent dye may be excited by installing a nano-opening smaller than wavelength of excited light at the flow cell substrate, and using evanescent light generated by irradiation of the excited light to the opening.

Control of the extension reaction is carried out by the control PC 426. The control PC 426 releases a shutter 445 of a UV laser 444. UV light illuminates a bottom surface of a substrate 432 via the same light pass as the above-described evanescent illumination. In the present Embodiment, the same optical system is used for irradiation of the UV laser 444 and excitation of the fluorescent dye. However, a illumination method itself of the UV laser is not especially limited to evanescent illumination, and it may also be an often used epi-illumination method or an oblique illumination method.

Accompanied by irradiation of UV laser, as already explained, the photo-protecting group dissociates and the extension reaction of nucleic acid proceeds automatically. When the extension reaction at a certain reaction field is completed, the control PC drives a servomotor 433 for driving the XY stage to transfer the next reaction field within a view of an objective lens 412. Still more, the control PC drives an auto focus device 427 under scattering light of the gold structure to focus on a fluorescent molecule at a flow cell wall surface. By repeating this operation, it becomes possible to repeat measurement of the real time base extension reaction sequentially, on 200 reaction fields arranged in the flow cell. In addition, it is possible to prepare a plurality of channels inside the substrate 432, and independently dispense the reaction solution for each channel using a dispensing unit 428. In addition, a waste liquid is accumulated in a waste liquid tank 429 through a scepter 431. In addition, in order to stabilize the real time base extension reaction, a temperature adjustment unit 450 is added.

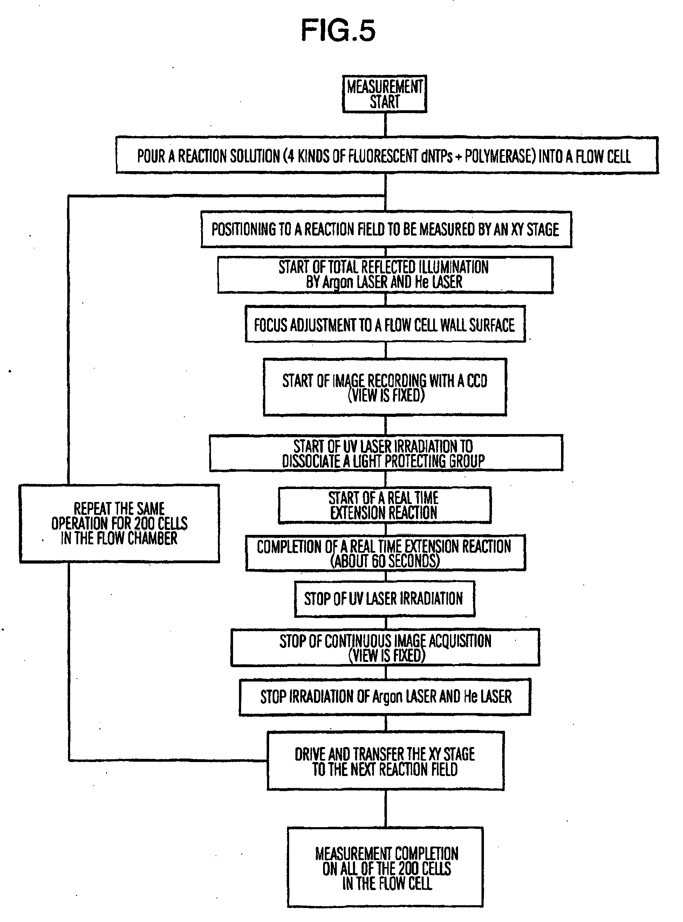

Fig. 5 shows a flow of the real time reaction. A reaction solution containing four kinds of fluorescent deoxynucleotides and a polymerase is poured into a flow cell. Next, an XY stage is driven to carry out positioning of a reaction field to be measured. Next, auto-focusing is carried out from a scattering image from the gold structure to a flow cell wall surface. After focusing onto the flow cell wall surface, acquiring of a continuous image of a CCD camera is initiated. By irradiation of UV laser, the photodegradation-active protecting group dissociates to initiate the real time extension reaction. After completion of the real time reaction for about 60 seconds, illumination of the UV laser is stopped to complete image acquisition. Still more, by stopping illumination of the Argon laser and the He-Ne laser, and moving the XY stage by a distance equal to clearance of the reaction fields, a reaction field, where the extension reaction is not yet started in the same flow cell, is positioned just under an objective lens. By repeating this operation 200 times for one flow cell, it is possible to realize the real time base extension reaction easily and conveniently without replacing the reaction solution. In addition, number of the reaction fields in the flow cell is not necessarily limited to 200 described in the present Embodiment. In addition, number of the flow cells formed at the substrate is also not especially limited.

It should be noted that, in the above-described Embodiment, a desired reaction field is arranged at an area irradiated with excited light, or an area irradiated with UV light, by stage drive, however, only the desired reaction field may be irradiated with the excited light or the UV light, without transferring the stage but by controlling an optical system.

### Reference Signs List

101, 102, 211, 212, 430, 431: scepter
103, 105, 107, 213, 432: substrate
104: PDMS
106, 201, 202, 203, 204: reaction field
108, 207: gold structure
109, 301, 303: oligo probe
206: mRNA
214: Alexa488-dCTP214
215: Cy3-dATP
216: Cy5-dCTP
217: Cy5.5-dCTP
220: caged compound
302: photodegradation-active protecting group
401: Argon laser
402: He-Ne laser
403, 404, 417, 440, 442, 443, 446: bandpass filter
405, 406, 414, 416, 221: dichroic mirror
407: ND filter
408: mirror
409: λ/4 wavelength plate
410, 415, 419, 422, 424: condenser lens
413: notch filter
418, 420, 423, 425: CCD camera
426: control PC
428: dispensing unit
429: waste liquid tank
433: servomotor for driving an XY stage
445: shutter
450: temperature adjustment unit

## Claims

1. A single molecule real time sequencer comprising:
nucleic acid probes (109, 205) having a photodegradable substance for inhibiting a nucleic acid extension reaction;
metal structures (108, 207) for generating a fluorescence increasing field arranged with said nucleic acid probes;
a reaction field area (106, 201-204) arranged with the metal structures;
a channel (205) installed with said reaction field areas;
a reagent supply mechanism (211, 212) for supplying a reagent to be utilized in the nucleic acid extension reaction to the channel;
an excited optical system (401, 402) for irradiating evanescent light to the desired reaction field area and detecting fluorescence generated; and
a reaction control optical system (426, 444, 445) for irradiating the light to decompose the photodegradable substance to the desired reaction field area;
wherein each nucleic acid probe is bonded to one of the metal structures.

2. A nucleic acid analyzer comprising:
a nucleic acid analysis device with nucleic acid probes (109, 205) adapted to hybridize with a target nucleic acid but not to promote a nucleic acid extension reaction unless UV light is irradiated,
metal structures (108, 207) for generating a fluorescence increasing field arranged with said nucleic acid probes;
a reaction field area (106, 201-204) arranged with said nucleic acid probes that hybridize with different target nucleic acids,
a nucleic acid analysis device having a channel (205) that is installed with said reaction field areas, and that is capable of retaining a reagent to be utilized in the nucleic acid reaction;
an excited optical system (401, 402) for irradiating the evanescent light to the desired reaction field area and detecting fluorescence generated;
a reaction control optical system (426, 444, 445) for irradiating the UV light to decompose the photodegradable substance to the desired reaction field area; and
a stage drive mechanism for moving relatively the nucleic acid analysis device to the excited optical system and the reaction control optical system, so as to irradiate evanescent light and UV light to the desired reaction field area;
wherein each nucleic acid probe is bonded to one of the metal structures.

3. A single molecule real time sequencing method comprising:
preparing nucleic acid probes having a photodegradable substance that inhibits a nucleic acid extension reaction, metal structures for generating a fluorescence increasing field arranged with said nucleic acid probes, and a channel installed with a plurality of reaction field areas arranged with said metal structures;
supplying a target nucleic acid and a reagent to be utilized in the nucleic acid extension reaction into the channel;
irradiating UV light to decompose the photodegradable substance to the desired reaction field area;
irradiating evanescent light to the desired reaction field area, and detecting fluorescence generated;
moving relatively the channel to the reaction control optical system for irradiating the UV light, and the excited optical system generating the evanescent light;
irradiating UV light to decompose the photodegradable substance to the different reaction field areas; and
irradiating evanescent light to the different reaction field areas and detecting fluorescence generated;
wherein each nucleic acid probe is bonded to one of the metal structures.

4. The invention of claim1 or 3, wherein the photodegradable substance is a photodegradable protecting group modified at the terminal of the nucleic acid probes.

5. The nucleic acid analyzer of claim 2, wherein the terminal of the nucleic acid probes is modified with the photodegradable protecting group modified.

6. The invention of claim 4 or 5, wherein the photodegradable protecting group is a 2-nitrobenzyl type, a decyl-phenacyl type or a coumarinylmethyl type.

7. The invention of any one of claims 1 to 3, wherein the reagent comprises four deoxynucleotides, dATP, dCTP, dTTP and dGTP, labeled with four-colored fluorescent dyes emitting each different fluorescence depending on the evanescent light, and an enzyme for carrying out the nucleic acid extension reaction.

8. The invention of any one of claims 1 to 3, wherein the excited optical system (401, 402) is adapted to illuminate by total reflection of visible light having a wavelength range of 420 nm to 800 nm, and to generate the evanescent light.

9. The invention of any one of claims 1 to 3, wherein the reaction control optical system irradiates UV light having a wavelength range of 250 nm to 400 nm.

10. The invention of any one of claims 1 to 3, wherein after the reaction control optical system (426, 444, 445) has irradiated light to the desired reaction area field, the channel (205) is moved to capture, in a view thereof, the reaction field area (106, 201-204) that has the different reaction control optical system.

## Patentansprüche

1. Einzelmolekül-Echtzeitsequenziereinrichtung, umfassend:
Nukleinsäuresonden (109, 205) mit einer fotoabbaubaren Substanz zur Hemmung einer Nukleinsäure-Erweiterungsreaktion;
Metallstrukturen (108, 207) zum Erzeugen eines mit den Nukleinsäuresonden angeordneten fluoreszenzsteigernden Feldes;
einen mit den Metallstrukturen angeordneten Reaktionsfeldbereich (106, 201-204);
einen mit den Reaktionsfeldbereichen eingerichteten Kanal (205);
einen Reagenszuführungsmechanismus (211, 212) zum Zuführen eines Reagens, das bei der Nukleinsäure-Erweiterungsreaktion verwendet werden soll, zum Kanal;
ein angeregtes optisches System (401, 402) zum Ausstrahlen von abklingendem Licht auf den erwünschten Reaktionsfeldbereich und zum Detektieren von erzeugter Fluoreszenz; und
ein optisches Reaktionssteuerungssystem (426, 444, 445) zum Ausstrahlen des Lichtes zum Abbauen der fotoabbaubaren Substanz auf dem erwünschten Reaktionsfeldbereich;
wobei jede Nukleinsäuresonde mit einer der Metallstrukturen verbunden ist.

2. Nukleinsäureanalyseeinrichtung, umfassend:
ein Nukleinsäureanalysegerät mit Nukleinsäuresonden (109, 205), die dazu ausgelegt sind, mit einer Zielnukleinsäure zu hybridisieren, aber nicht eine Nukleinsäure-Erweiterungsreaktion zu fördern, außer wenn UV-Licht ausgestrahlt wird,
Metallstrukturen (108, 207) zum Erzeugen eines mit den Nukleinsäuresonden angeordneten fluoreszenzsteigernden Feldes;
einen Reaktionsfeldbereich (106, 201-204), der mit den Nukleinsäuresonden angeordnet ist, die mit verschiedenen Zielnukleinsäuresonden hybridisieren,
ein Nukleinsäureanalysegerät mit einem Kanal (205), der mit den Reaktionsfeldbereichen eingerichtet ist und der dazu in der Lage ist, ein bei der Nukleinsäurereaktion zu verwendendes Reagens zu verwahren;
ein angeregtes optisches System (401, 402) zum Ausstrahlen des abklingenden Lichtes auf den erwünschten Reaktionsfeldbereich und zum Detektieren von erzeugter Fluoreszenz;
ein optisches Reaktionssteuerungssystem (426, 444, 445) zum Ausstrahlen des UV-Lichtes zum Abbauen der fotoabbaubaren Substanz auf dem erwünschten Reaktionsfeldbereich und
einen Gestellantriebsmechanismus zum relativen Bewegen des Nukleinsäureanalysegeräts zu dem angeregten optischen System und dem optischen Reaktionssteuerungssystem, um so abklingendes Licht und UV-Licht auf den erwünschten Reaktionsfeldbereich auszustrahlen;
wobei jede Nukleinsäuresonde mit einer der Metallstrukturen verbunden ist.

3. Einzelmolekül-Echtzeitsequenzierungsverfahren, umfassend die folgenden Schritte:
Vorbereiten von Nukleinsäuresonden mit einer fotoabbaubaren Substanz, die eine Nukleinsäure-Erweiterungsreaktion hemmt, von Metallstrukturen zum Erzeugen eines mit den Nukleinsäuresonden angeordneten fluoreszenzsteigernden Feldes und von einem Kanal, der mit mehreren Reaktionsfeldbereichen eingerichtet ist, die mit den Metallstrukturen angeordnet sind;
Zuführen einer Zielnukleinsäure und eines bei der Nukleinsäure-Erweiterungsreaktion zu verwendenden Reagens in den Kanal;
Ausstrahlen von UV-Licht zum Abbauen der fotoabbaubaren Substanz auf den erwünschten Reaktionsfeldbereich;
Ausstrahlen von abklingendem Licht auf den erwünschten Reaktionsfeldbereich und Detektieren von erzeugter Fluoreszenz;
relatives Bewegen des Kanals zum optischen Reaktionssteuerungssystem zum Ausstrahlen des UV-Lichts und zum angeregten optischen System, das das abklingende Licht erzeugt;
Ausstrahlen von UV-Licht zum Abbauen der fotoabbaubaren Substanz auf die verschiedenen Reaktionsfeldbereiche und
Ausstrahlen von abklingendem Licht auf die verschiedenen Reaktionsfeldbereiche und Detektieren von erzeugter Fluoreszenz;
wobei jede Nukleinsäuresonde mit einer der Metallstrukturen verbunden ist.

4. Erfindung nach Anspruch 1 oder 3, wobei die fotoabbaubare Substanz eine fotoabbaubare Schutzgruppe ist, die am Anschluss der Nukleinsäuresonden modifiziert worden ist.

5. Nulkeinsäureanalyseeinrichtung nach Anspruch 2, wobei der Anschluss der Nukleinsäuresonden mit der modifizierten fotoabbaubaren Schutzgruppe modifiziert wird.

6. Erfindung nach Anspruch 4 oder 5, wobei die fotoabbaubare Schutzgruppe vom 2-Nitrobenzyl-, Decyl-Phenacyl- oder Cumarinylmethyl-Typ ist.

7. Erfindung nach einem der Ansprüche 1 bis 3, wobei das Reagens vier Desoxynukleotide, dATP, dCTP, dTTP und dGTP, mit vierfarbigen Fluoreszenz-Färbern, die jeweils verschiedene Fluoreszenz in Abhängigkeit vom abklingenden Licht emittieren, markiert, und ein Enzym zum Ausführen der Nukleinsäure-Erweiterungsreaktion umfasst.

8. Erfindung nach einem der Ansprüche 1 bis 3, wobei das angeregte optische System (401, 402) dazu ausgelegt ist, mittels Totalreflektion von sichtbarem Licht mit einem Wellenlängenbereich von 420 nm bis 800 nm zu leuchten und das abklingende Licht zu erzeugen.

9. Erfindung nach einem der Ansprüche 1 bis 3, wobei das optische Reaktionssteuerungssystem UV-Licht mit einem Wellenlängenbereich von 250 nm bis 400 nm ausstrahlt.

10. Erfindung nach einem der Ansprüche 1 bis 3, wobei der Kanal (205), nachdem das optische Reaktionssteuerungssystem. (426, 444, 445) Licht auf den erwünschten Reaktionsfeldbereich ausgestrahlt hat, bewegt wird, um in einer Ansicht davon den Reaktionsfeldbereich (106, 201-204) zu erfassen, der zum unterschiedlichen optischen Reaktionsssteuerungssystem gehört.

## Revendications

1. Séquenceur en temps réel de molécules simples comportant :
des sondes d'acide nucléique (109, 205) ayant une substance photodégradable qui inhibe une réaction d'extension d'acide nucléique,
des structures métalliques (108, 207) pour générer un champ augmentation de fluorescence agencé avec lesdites sondes d'acide nucléique,
une zone de champ de réaction (106, 201-204) agencée avec les structures métalliques,
un canal (205) installé avec lesdites zones de champ de réaction,
un mécanisme d'alimentation en réactif (211, 212) pour délivrer un réactif à utiliser dans la réaction d'extension d'acide nucléique dans le canal,
un système optique excité (401, 402) pour irradier une lumière évanescente dans la zone de champ de réaction voulue et détecter la fluorescence générée, et
un système optique de commande de réaction (426, 444, 445) pour irradier la lumière afin de décomposer la substance photodégradable dans la zone de champ de réaction voulue,
dans lequel chaque sonde d'acide nucléique est liée à l'une des structures métalliques.

2. Analyseur d'acide nucléique comportant :
un dispositif d'analyse d'acide nucléique ayant des sondes d'acide nucléique (109, 205) adaptées pour s'hybrider avec un acide nucléique cible mais pas pour favoriser une réaction d'extension d'acide nucléique sauf si une lumière UV est irradiée,
des structures métalliques (108, 207) pour générer un champ d'augmentation de fluorescence agencé avec lesdites sondes d'acide nucléique,
une zone de champ de réaction (106, 201-204) agencée avec lesdites sondes d'acide nucléique qui s'hybrident avec différents acides nucléiques cibles,
un dispositif d'analyse d'acide nucléique ayant un canal (205) qui est installé avec lesdites zones de champ de réaction, et qui est capable de conserver un réactif à utiliser dans la réaction d'acide nucléique,
un système optique excité (401, 402) pour irradier la lumière évanescente dans la zone de champ de réaction voulue et détecter la fluorescence générée,
un système optique de commande de réaction (426, 444, 445) pour irradier la lumière UV afin de décomposer la substance photodégradable dans la zone de champ de réaction voulue, et
un mécanisme d'entraînement à étage pour déplacer relativement le dispositif d'analyse d'acide nucléique dans le système optique excité et le système optique de commande de réaction, de manière à irradier une lumière évanescente et une lumière UV dans la zone de champ de réaction voulue,
dans lequel chaque sonde d'acide nucléique est liée à l'une des structures métalliques.

3. Procédé de séquençage en temps réel de molécules simples comportant les étapes consistant à :
préparer des sondes d'acide nucléique ayant une substance photodégradable qui inhibe une réaction d'extension d'acide nucléique, des structures métalliques pour générer un champ augmentation de fluorescence avec lesdites sondes d'acide nucléique, et un canal installé avec une pluralité de zones de champ de réaction disposées avec lesdites structures métalliques,
délivrer un acide nucléique cible et un réactif à utiliser dans la réaction d'extension d'acide nucléique dans le canal,
irradier une lumière UV pour décomposer la substance photodégradable dans la zone de champ de réaction voulue,
irradier une lumière évanescente dans la zone de champ de réaction voulue, et détecter une fluorescence générée,
déplacer relativement le canal dans le système optique de commande de réaction pour irradier la lumière UV, et le système optique généré générant la lumière évanescente,
irradier une lumière UV pour décomposer la substance photodégradable dans les différentes zones de champ de réaction, et
irradier une lumière évanescente dans les différentes zones de champ de réaction et détecter la fluorescence générée,
dans lequel chaque sonde d'acide nucléique est liée à l'une des structures métalliques.

4. Invention selon la revendication 1 ou 3, dans laquelle la substance photodégradable est un groupe protecteur photodégradable modifié au niveau de la borne des sondes d'acide nucléique.

5. Analyseur d'acide nucléique selon la revendication 2, dans lequel la borne des sondes d'acide nucléique est modifiée avec le groupe protecteur photodégradable modifié.

6. Invention selon la revendication 4 ou 5, dans lequel le groupe protecteur photodégradable est un type 2-nitrobenzyle, un type décyl-phénacyle ou un type coumarin-ylméthyle.

7. Invention selon l'une quelconque des revendications 1 à 3, dans laquelle le réactif comporte quatre désoxynucléotides, dATP, dCTP, dTTP et dGTP, marqués avec des colorants fluorescents quadricolores émettant chacun une fluorescence différente en fonction de la lumière évanescente, et une enzyme pour réaliser la réaction d'extension d'acide nucléique.

8. Invention selon l'une quelconque des revendications 1 à 3, dans laquelle le système optique excité (401, 402) est adapté pour illuminer par réflexion totale de lumière visible ayant une plage de longueur d'onde de 420 nm à 800 nm, et pour générer la lumière évanescente.

9. Invention selon l'une quelconque des revendications 1 à 3, dans laquelle le système optique de commande de réaction irradie une lumière UV ayant une plage de longueur d'onde de 250 nm à 400 nm.

10. Invention selon l'une quelconque des revendications 1 à 3, dans laquelle une fois que le système optique de commande de réaction (426, 444, 445) a irradié une lumière dans le champ de zone de réaction voulu, le canal (205) est déplacé pour capturer, dans une vue correspondante, la zone de champ de réaction (106, 201-204) qui a le système optique de commande de réaction différent.
